# EUROPEAN PATENT SPECIFICATION

(11) **EP 0 223 385 B1**
(45) Date of publication and mention of the grant of the patent: **10.03.1993**
(21) Application number: 86307720.2
(22) Date of filing: 07.10.1986
(51) Int. Cl.: C07D 451/12, C07D 451/14, A61K 31/46, A61K 31/435

(54) **8-Azabicyclo[3,2,1]octane and 9-azabicyclo[3,3,1]nonane derivatives**
8-Azabicyclo[3,2,1]octan- und 9-Azabicyclo[3,3,1]nonanderivate
8-Azabicyclo[3,2,1]octane et 9-azabicyclo[3,3,1]nonane dérivés

(30) Priority: 21.10.1985 GB 8525913
(43) Date of publication of application: 27.05.1987
(73) Proprietor: BEECHAM GROUP PLC, Brentford, Middlesex TW8 9EP (GB)
(72) Inventor: King, Francis David, c/o SmithKline Beecham, Harlow, Essex CM19 5AD (GB)
(74) Representative: Tocher, Pauline

(56) References cited:
- WO-A-84/00166
- WO-A-85/01048

## Description

This invention relates to novel compounds having useful pharmacological properties, to pharmaceutical compositions containing them, to a process and intermediates for their preparation, and to their use as pharmaceuticals.

UK Patent Applications, GB 2100259A and 2125398A describe benzoates and benzamides having an azabicyclic side chain and possessing 5-HT (5-Hydroxytryptamine) antagonist activity. WO-A-85/01048 describes 3-indolecarboxylic acid (8-azabicyclo[3.2.1]octyl and 9-azabicyclo[3.3.1]nonyl)esters and amides having 5HT-antagonist activity.

A class of novel, structurally distinct compounds has now been discovered. These compounds have 5-HT antagonist activity and/or gastric motility enhancing activity.

Accordingly, the present invention provides a compound of formula (I), or a pharmaceutically acceptable salt thereof: 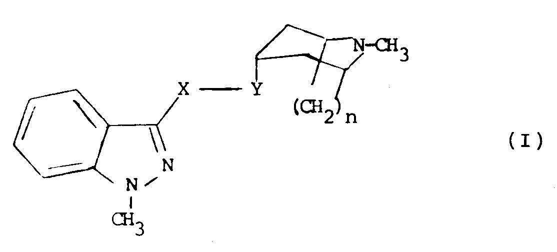
wherein
X is CO and Y is NH or O; and
n is 2 or 3.

Preferably Y is NH and n is 3.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (I) are usually acid addition salts with acids such as hydrochloric, hydrobromic, phosphoric, sulphuric, citric, tartaric, lactic and acetic acid.

Preferably the acid addition salt is the hydrochloride salt.

Examples of pharmaceutically acceptable salts include quaternary derivatives of the compounds of formula (I) such as the compounds quaternised by compounds R-T wherein R is C₁₋₆ alkyl, phenyl-C₁₋₆ alkyl or C₅₋₇ cycloalkyl, and T is a radical corresponding to an anion of an acid. Suitable examples of R include methyl, ethyl and n- and iso-propyl; and benzyl and phenethyl. Suitable examples of T include halide such as chloride, bromide and iodide.

Examples of pharmaceutically acceptable salts of the compounds of formula (I) also form internal salts such as pharmaceutically acceptable N-oxides.

The compounds of the formula (I) and their pharmaceutically acceptable salts, (including quaternary derivatives and N-oxides) may also form pharmaceutically acceptable solvates, such as hydrates, which are included whenever such compounds and salts are herein referred to.

It will of course be realised that the compounds of the formula (I) have chiral or prochiral centres and thus are capable of existing in a number of stereoisomeric forms including enantiomers. The invention extends to each of these stereoisomeric forms (including enantiomers), and to mixtures thereof (including racemates). The different stereoisomeric forms may be separated one from the other by the usual methods.

The invention also provides a process for the preparation of a compound of formula (I), or a pharmaceutically acceptable salt thereof, which process comprises reacting a compound of formula (V): 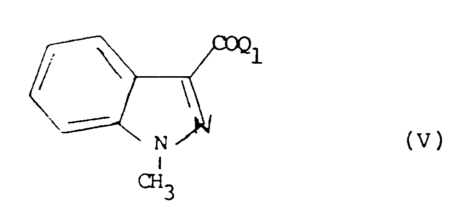
with a compound of formula (VI): 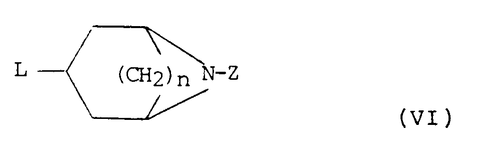
wherein
Q₁ is a group displaceable by a nucleophile, L is NH₂ or OH or a reactive derivative thereof, Z is methyl or a group convertible to methyl; and the remaining variables are as hereinbefore defined; and thereafter optionally forming a pharmaceutically acceptable salt of the resultant compound of formula (I).

Examples of leaving groups Q₁, displaceable by a nucleophile include halogen such as chloro and bromo, hydroxy, carboxylic acyloxy such as C₁₋₄ alkanoyloxy or C₁₋₄ alkoxycarbonyloxy and activated hydrocarbyloxy such as pentachlorophenoxy.

If a group Q₁ is a halide, then the reaction is preferably carried out at non-extreme temperatures in an inert non-hydroxylic solvent, such as benzene, dichloromethane, toluene, diethyl ether, THF (tetrahydrofuran) or DMF (dimethylformamide). It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate. Temperatures of 0^{o}-100^{o}C, in particular 10-80^{o}C are suitable.

If a group Q₁ is hydroxy, then the reaction is generally carried out in an inert non-hydroxylic solvent, such as dichloromethane, THF or DMF optionally in the presence of a dehydrating catalyst, such as a carbodiimide, for example dicyclohexylcarbodiimide. The reaction may be carried out at any non-extreme temperature, such as -10 to 100^{o}C, for example, 0 to 80^{o}C. Generally, higher reaction temperatures are employed with less active compounds whereas lower temperatures are employed with the more active compounds.

If a group Q₁ is carboxylic acyloxy, then the reaction is preferably carried in substantially the same manner as the reaction when Q₁ is halide. Suitable examples of acyloxy leaving groups include C₁₋₄ alkanoyloxy and C₁₋₄ alkoxycarbonyloxy, in which case the reaction is preferably carried out in an inert solvent, such as methylene chloride, at a non-extreme temperature for example ambient temperatures in the presence of an acid acceptor, such as triethylamine. C₁₋₄ alkoxycarbonyloxy leaving groups may be generated in situ by treatment of the corresponding compound wherein Q₁ is hydroxy with a C₁₋₄ alkyl chloroformate.

If a group Q₁ is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

When L is OH or a reactive derivative thereof, the reactive derivative is often a salt, such as the lithium salt.

Pharmaceutically acceptable salts of the compounds of this invention may be formed conventionally.

The salts may be formed for example by reaction of the base compound of formula (I) with a pharmaceutically acceptable organic or inorganic acid.

Z may be optionally substituted benzyl. Such benzyl groups may, for example, be removed, by conventional transition metal catalysed hydrogenolysis to give compounds of the formula (VII): 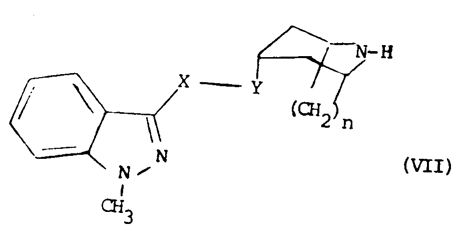
wherein the variables are as defined in formula (I).

This invention also provides a further process for the preparation of a compound of the formula (I) which comprises N-methylating a compound of formula (VII), and optionally forming a pharmaceutically acceptable salt, of the resulting compound of the formula (I).

This may be achieved by reaction of the compound of formula (VII) with a compound CH₃Q₂ wherein Q₂ is a leaving group. Suitable values for Q₂ include groups displaced by nucleophiles such as Cl, Br, I, OSO₂CH₃ or OSO₂C₆H₄pCH₃. Favoured values for Q₂ include Cl, Br and I.

The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above.

Alternatively, 'N-methylation' may be effected under conventional reductive alkylation conditions. It is often convenient to prepare the corresponding compound where the methyl group is replaced by esterified carboxyl. Such compounds may then be reduced using a strong reductant such as lithium aluminium hydride to the corresponding compound of formula (V).

The compounds of formula (V) and (VI) are known or are preparable analogously to, or routinely from, known compounds.

It will be realised that in the compound of the formula (I) the -X-Y-linkage has an endo orientation with respect to the ring of the bicyclic moiety to which it is attached. A mixture of endo and exo isomers of the compound of the formula (I) may be synthesised non-stereospecifically and the desired isomer separated conventionally therefrom e.g. by chromatography; or alternatively the endo isomer may if desired by synthesised from the corresponding isomer of the compound of the formula (VI).

The compounds of the present invention are 5-HT antagonists and it is thus believed may generally be used in the treatment or prophylaxis of migraine, cluster headaches and trigeminal neuralgia; and also as anti-emetics, in particular that of preventing vomiting and nausea associated with cancer therapy, and motion sickness. Examples of such cancer therapy include that using cytotoxic agents, such as cisplatin, doxorubicin and cyclophosphamide, particularly cisplatin; and also radiation treatment. Compounds which are 5-HT antagonists may also be of potential use in the treatment of CNS disorders such as anxiety and psychosis; arrhythmia, obesity and irritable bowel syndrome.

The compounds of the present invention also have gastric motility enhancing activity, useful in the treatment of disorders such as retarded gastric emptying, dyspepsia, flatulence, oesophagal reflux and peptic ulcer.

The invention also provides a pharmaceutical composition comprising a compound of formula (I), or a pharmaceutically acceptable salt thereof, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment or prophylaxis of migraine, cluster headache, trigeminal neuralgia and/or emesis in mammals, such as humans, which comprises the administration to the mammal of an effective amount of a compound of the formula (I) or a pharmaceutically acceptable salt thereof.

An amount effective to treat the disorders hereinbefore described depends on the relative efficacies of the compounds of the invention, the nature and severity of the disorder being treated and the weight of the mammal. However, a unit dose for a 70kg adult will normally contain 0.5 to 1000mg for example 1 to 500mg, of the compound of the invention. Unit doses may be administered once or more than once a day, for example, 2, 3 or 4 times a day, more usually 1 to 3 times a day, that is in the range of approximately 0.001 to 50 mg/kg/day, more usually 0.002 to 25 mg/kg/day.

No adverse toxicological effects are indicated at any of the aforementioned dosage ranges.

The invention also provides a compound of formula (I) or a pharmaceutically acceptable salt thereof for use as an active therapeutic substance, in particular for use in the treatment of migraine, cluster headache, trigeminal neuralgia and/or emesis.

The following Examples illustrate the preparation of compounds of formula (I).

N.B. Nomenclature is based on Chemical Abstracts Index Guide 1977 published by the American Chemical Society.

### Preparation

### 3-Indazolecarboxylic acid (endo-8-methyl-8-azabiclo[3.2.1]oct-3-yl)ester

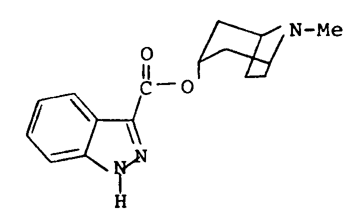

A solution of tropine (0.45g) and KBu^{t}O (0.36g) in amine-free DMF (50ml) was stirred at room temperature for 30min. The more volatile t-butanol was removed by rotary evaporation and the residual solution treated with diindazolo[2,3-a 2',3'-d]pyrazine-7,14-dione (0.2g). After heating to 120^{o} for 2h, the reaction mixture was cooled, evaporated to dryness and the residue treated with saturated NaHCO₃ solution (50ml). The pH was adjusted to ca.8 with acetic acid and the product extracted into CHCl₃ (3 x 100ml). The organic extracts were dried (Na₂SO₄), evaporated to dryness and the residue triturated with diethylether to give the title compound (0.16g) mp 234-5^{o} (dec).
- ¹H NMR (270MHz, d₆-DMSO): δ 13.5 (1H, brs)
8.18 (1H, d)
7.60 (1H, d)
7.39 (1H, t)
7.28 (1H, t)
5.31 (1H, t)
3.23 (2H, brs)
2.36 (3H, s)
2.45-1.90 (8H, m)

### Example 1

### 1-Methyl-3-indazolecarboxylic acid (endo-8-methyl-8-azabicyclo[3,2,1]oct-3-yl)ester monohydrochloride

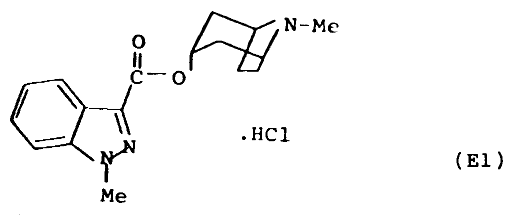

Following the procedure outlined in the Preparation, the potassium salt of tropine (0.37g) was reacted with 1-methyl3-indazolcarboxylic acid chloride (0.21g) to give, after treatment with ethanolic hydrogen chloride, the title compound (E1) (0.21g) m.p. 257-260^{o}C.
- ¹H NMR (79.5 MHz, CDCl₃): δ 8.30-8.10 (m, 1H)
7.60-7.20 (m, 3H)
5.55-5.30 (m, 1H)
4.18 (s, 3H)
4.00-3.70 (m, 2H)
3.40-2.00 (m, 11H including 2.83, s, 3H)

### Example 2

### N-(Endo-9-methyl-9-azabicyclo[3,3,1]non-3-yl)-1-methylindazole-3-carboxamide monohydrochloride

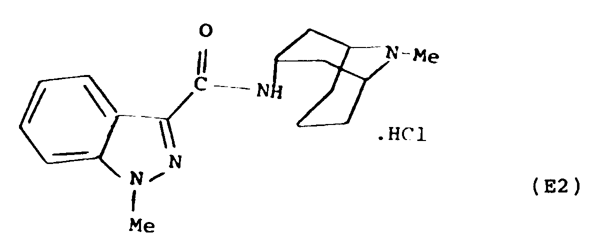

A stirred solution of 1-methylindazole-3-carboxylic acid chloride (0.77g) in dichloromethane (50ml) was treated with a solution of endo-9-methyl-9-azabicyclo[3,3,1]nonan-3-amine (0.7g) and triethylamine (0.7ml) in dichloromethane (30ml). After 2h, the reaction mixture was washed with saturated aqueous NaHCO₃ (100ml) and dried (K₂CO₃). The oil remaining after evaporation of the solvent was purified by column chromatography (TLC-alumina, CHCl₃) and treated with hydrogen chloride to give the title compound E2. m.p. 290-2^{o}C.
- ¹H NMR (270 MHz, CDCl₃): δ 8.30 (d, 1H)
7.50-7.20 (m, 4H)
4.80-4.50 (m, 1H)
4.12 and 4.10 (2-s, 3H)
3.75-3.55 (m, 2H)
2.99 and 2.91 (2-s, 3H)
2.82-2.40 (m, 4H)
2.20-2.00 (m, 2H)
1.90-1.60 (m, 4H)

### Pharmacology

### Antagonism of the von Bezold-Jarisch reflex

The compounds were evaluated for antagonism of the von Bezold-Jarisch reflex evoked by 5-HT in the anaesthetised rat according to the following method:

Male rats 250-350g, were anaesthetised with urethane (1.25g/kg intraperitoneally) and blood pressure and heart rate recorded as described by Fozard J.R. et al., J. Cardiovasc. Pharmacol. 2, 229-245 (1980). A submaximal dose of 5-HT (usually 6µg/kg) was given repeatedly by the intravenous route and changes in heart rate quantified. Compounds were given intravenously and the concentration required to reduce the 5 HT-evoked response to 50% of the control response (ED₅₀) was then determined.

The results were as follows.

| Compound No. | ED₅₀ (mg/kg) |
|---|---|
| 1 | 0.0014 |
| 2 | 0.0007 |

## Claims (Claims for the following Contracting State(s): GR)

1. A compound of formula (I), or a pharmaceutically acceptable salt thereof: 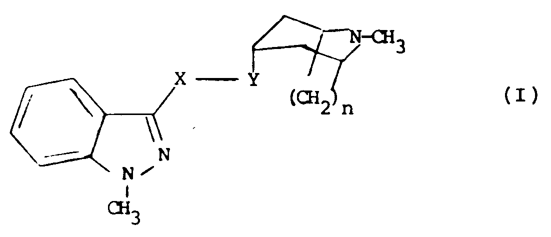 wherein
X is CO and Y is NH or O; and
n is 2 or 3.

2. A compound according to claim 1 wherein Y is NH.

3. A compound according to claim 1 or 2 wherein n is 3.

4. 1-Methyl-3-indazolecarboxylic acid(endo-8-methyl-8-azabicyclo[3,2,1]oct-3yl)ester, or a pharmaceutically acceptable salt thereof.

5. N-(Endo-9-methyl-9-azabicyclo[3,3,1]non-3-yl)-1-methyl-indazole-3-carboxamide, or a pharmaceutically acceptable salt thereof.

6. Use of a compound of formula (I) according to any one of claims 1 to 5, or a pharmaceutically acceptable salt thereof, in the manufacture of a medicament for the treatment of migraine, cluster headache, trigeminal neuralgia and/or emesis in mammals.

## Claims (Claims for the following Contracting State(s): AT, ES)

1. A process for the preparation of a compound of formula (I), or a pharmaceutically acceptable salt thereof: 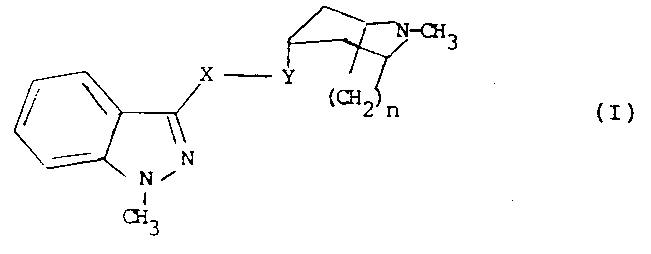 wherein
X is CO and Y is NH or O; and
n is 2 or 3; which process comprises reacting a compound of formula (V): 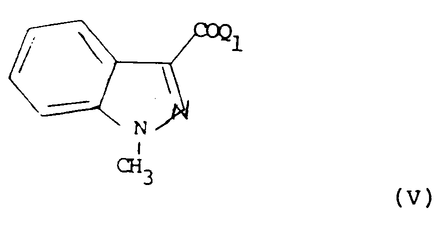 with a compound of formula (VI): 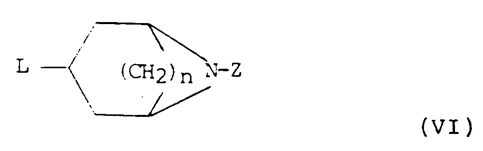 wherein
Q₁ is a group displaceable by a nucleophile, L is NH₂ or OH or a reactive derivative thereof, Z is methyl or a group convertible to methyl; and the remaining variables are as hereinbefore defined; and thereafter optionally forming a pharmaceutically acceptable salt of the resultant compound of formula (I).

2. A process according to claim 1 wherein Y is NH.

3. A process according to claim 1 or 2 wherein n is 3.

4. A process according to claim 1 for the preparation of 1-Methyl-3-indazolecarboxylic acid(endo-8- methyl-8-azabicyclo[3,2,1]oct-3yl)ester, or a pharmaceutically acceptable salt thereof.

5. A process according to claim 1 for the preparation of N-(Endo-9-methyl-9-azabicyclo [3,3,1]non-3-yl)- 1-methyl-indazole-3-carboxamide, or a pharmaceutically acceptable salt thereof.

6. Use of a compound of formula (I) according to any one of claims 1 to 5, or a pharmaceutically acceptable salt thereof, in the manufacture of a medicament for the treatment of migraine, cluster headache, trigeminal neuralgia and/or emesis in mammals.

## Patentansprüche (Patentansprüche für folgende(n) Vertragsstaat(en): GR)

1. Verbindung der Formel (I) oder ein pharmazeutisch verträgliches Salz davon: 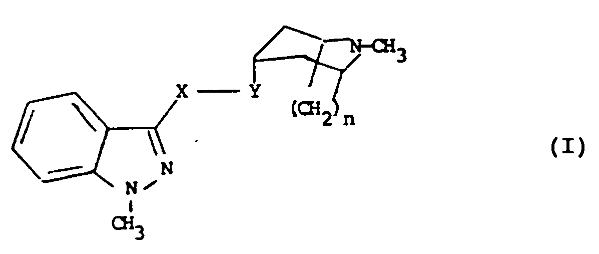 in der
X die Gruppe CO bedeutet und Y die Gruppe NH oder O darstellt, und
n 2 oder 3 ist.

2. Verbindung nach Anspruch 1, wobei Y die Gruppe NH bedeutet.

3. Verbindung nach Anspruch 1 oder 2, wobei n 3 ist.

4. 1-Methyl-3-indazolcarbonsäure(endo-8-methyl-8-azabicyclo[3,2,1]oct-3yl)ester oder ein pharmazeutisch verträgliches Salz davon.

5. N-(Endo-9-methyl-9-azabicyclo[3,3,1]non-3-yl)-1-methylindazol-3-carboxamid oder ein pharmazeutisch verträgliches Salz davon.

6. Verwendung einer Verbindung der Formel I nach einem der Ansprüche 1 bis 5 oder eines pharmazeutisch verträglichen Salzes davon zur Herstellung eines Arzneimittels für die Behandlung von Migräne, clusterartigem Kopfschmerz, trigeminaler Neuralgie und/oder Emesie bei Säugern.

## Patentansprüche (Patentansprüche für folgende(n) Vertragsstaat(en): AT, ES)

1. Verfahren zur Herstellung einer Verbindung der Formel (I) oder eines pharmazeutisch verträglichen Salzes davon: 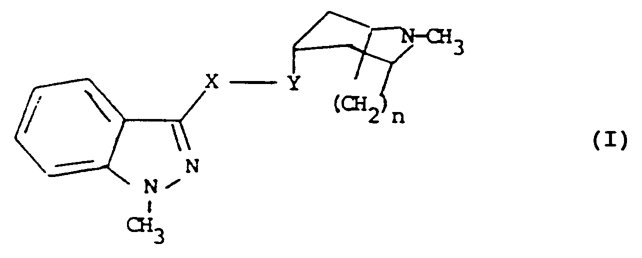 in der
X die Gruppe CO bedeutet und Y die Gruppe NH oder O bedeutet; und
n 2 oder 3 ist; umfassend die Umsetzung einer Verbindung der Formel (V): 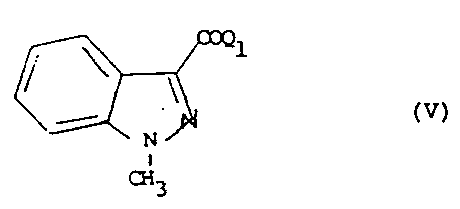 mit einer Verbindung der Formel (VI): 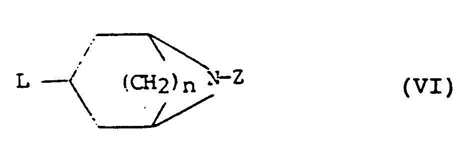 in der
Q₁ eine durch ein Nucleophil ersetzbare Gruppe ist, L die Gruppe NH₂ oder OH oder ein reaktives Derivat davon bedeutet, Z eine Methylgruppe oder eine in eine Methylgruppe umwandelbare Gruppe bedeutet, und die übrigen Variablen wie vorstehend definiert sind und anschließend gegebenenfalls Herstellen eines pharmazeutisch verträglichen Salzes der erhaltenen Verbindung der Formel (I).

2. Verfahren nach Anspruch 1, wobei Y die Gruppe NH bedeutet.

3. Verfahren nach Anspruch 1 oder 2, wobei n 3 ist.

4. Verfahren nach Anspruch 1 zur Herstellung von 1-Methyl-3-indazolcarbonsäure(endo-8-methyl-8-azabicyclo[3,2,1]oct-3yl)ester oder eines pharmazeutisch verträglichen Salzes davon.

5. Verfahren nach Anspruch 1 zur Herstellung von N-(Endo-9-methyl-9-azabicyclo[3,3,1]non-3-yl)-1-methyl-indazol-3-carboxamid oder eines pharmazeutisch verträglichen Salzes davon.

6. Verwendung einer Verbindung der Formel (I) nach einem der Ansprüche 1 bis 5 oder eines pharmazeutisch verträglichen Salzes davon zur Herstellung eines Arzneimittels für die Behandlung von Migräne, clusterartigem Kopfschmerz, trigeminaler Neuralgie und/oder Emesie bei Säugern.

## Revendications (Revendications pour l'(les) Etat(s) contractant(s) suivant(s): GR)

1. Composé de formule (I) ou sel de celui-ci acceptable du point de vue pharmaceutique: 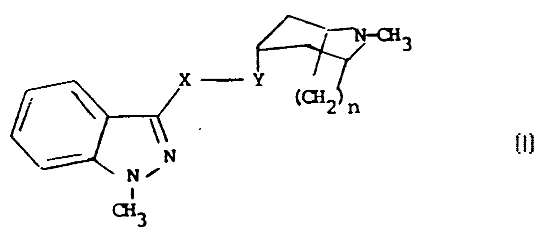 dans laquelle :
X est le radical CO et Y est le radical NH ou un atome d'oxygène, et n est 2 ou 3.

2. Composé suivant la revendication 1, dans lequel Y et le radical NH.

3. Composé suivant les revendications 1 ou 2, dans lequel n est 3.

4. Composé suivant la revendication 1, qui est l'ester endo-8-méthyl-8-azabicyclo-[3,2,1]-oct-3-ylique de l'acide 1-méthyl-3-indazolecarboxylique ou un sel de celui-ci acceptable du point de vue pharmaceutique.

5. Composé suivant la revendication 1, qui est le N-(endo-9-méthyl-9-azabicylo[3,3,1]non-3-yl)-1-méthyl-indazole-3-carboxamide ou un sel de celui-ci acceptable du point de vue pharmaceutique.

6. Utilisation d'un composé de formule (I) suivant l'une quelconque des revendications 1 à 5, ou d'un sel de celui-ci acceptable du point de vue pharmaceutique, dans a fabrication d'un médicament pour le traitement de la migraine, du névralgisme facial, de la névralgie faciale du trijumeau et/ou des vomissements chez les mammifères.

## Revendications (Revendications pour l'(les) Etat(s) contractant(s) suivant(s): AT, ES)

1. Procédé pour la préparation d'un composé de formule (I) ou d'un sel de celui-ci acceptable du point de vue pharmaceutique : 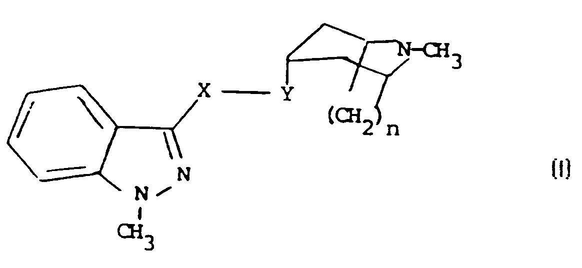 dans laquelle :
X est le radical CO et Y est le radical NH ou un atome d'oxygène, et n est 2 ou 3;
lequel procédé comprend la réaction d'un composé de formule (V): 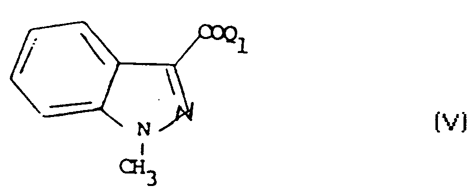 avec un compose' de formule (VI): 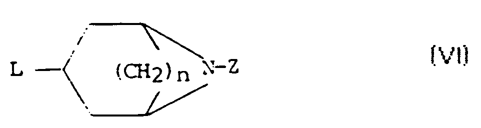 dans lesquelles:
Q₁ est un groupe pouvant être déplacé par un nucléophile, L est un groupe NH₂ ou OH ou un dérivé réactif de ceux-ci, Z est un groupe méthyle ou un groupe pouvant être converti en groupe méthyle les autres variables étant telles que définies plus haut; et ensuite, la formation éventuelle d'un sel acceptable du point de vue pharmaceutique du composé résultant de formule (I).

2. Procédé suivant la revendication 1, dans lequel Y est le radical NH.

3. Procédé suivant les revendications 1 ou 2, dans lequel n est 3.

4. Procédé suivant la revendication 1, pour la préparation de l'ester endo-8-méthyl-8-azabicyclo-[3,2,1]-oct-3-ylique de l'aide 1-méthyl-3-indazolecarboxylique ou d'un sel de celui-ci acceptable du point de vue pharmaceutique.

5. Procédé suivant la revendication 1, pour la préparation du N-(endo-9-méthyl-9-azabicyclo[3,3,1]non-3-yl)-1-méthyl-indazole-3-carboxamide ou d'un sel de celui-ci acceptable du point de vue pharmaceutique.

6. Utilisation d'un composé de formule (I) suivant l'une quelconque des revendications 1 à 5, ou d'un sel de celui-ci acceptable du point de vue pharmaceutique, dans la fabrication d'un médicament pour le traitement de la migraine, du névralgisme facial, de la névralgie faciale du trijumeau et/ou des vomissements chez les mammifères.
